# EUROPEAN PATENT SPECIFICATION

(11) **EP 0 455 767 B1**
(45) Date of publication and mention of the grant of the patent: **25.01.1995**
(21) Application number: 90916729.8
(22) Date of filing: 23.11.1990
(51) Int. Cl.: A61F 6/14

(54) **PROCESS FOR THE MANUFACTURE OF A CARRIER OR IUD PROVIDED WITH A PULLING ELEMENT, AND A CARRIER OR IUD PROVIDED WITH A PULLING ELEMENT**
TRÄGER- ODER INTRAUTERINE VORRICHTUNG MIT EINER ZUGEINRICHTUNG SOWIE VERFAHREN ZUR HERSTELLUNG DAVON
PROCEDE DE FABRICATION D'UN SUPPORT OU D'UN STERILET POURVU D'UN ELEMENT A TIRET ET SUPPORT OU STERILET AVEC ELEMENT A TIRET AINSI PRODUITS

(30) Priority: 23.11.1989 NL 8902896
(43) Date of publication of application: 13.11.1991
(73) Proprietor: METALLICOM A.G., CH-Fribourg (CH)
(72) Inventor: CUSTERS, Hilaire, Adolf, Franciscus, Marie, NL-6865 CG Doorwerth (NL); KURZ, Karl-Heinz, D-4000 Düsseldorf (DE)
(74) Representative: Morel, Christiaan F., Ir.Dr.
(86) International application number: EP9002014
(87) International publication number: WO9107934

(56) References cited:
- FR-A- 2 431 286
- GB-A- 981 389
- US-A- 3 407 806
- US-A- 3 842 826
- US-A- 4 094 313

## Description

The invention relates to a process for the manufacture of an intra uterine device or IUD made of plastic material provided with a pulling element, and a middle projection involving that first a thread is placed in the injection mold for the manufacture of the IUD in the lengthwise direction of the middle projection and that subsequently the mold is closed and the IUD is produced by injection molding, after which the IUD is removed from the mold and to a carrier for a contra-ceptive of the coil type known by the name of IUD, which carrier comprises an essentially T-shaped plastic element with a middle leg on which a so called coil is wound, while cross pieces of the T-shape serve to hold the IUD in place after the IUD has been introduced into the uterus of a user, and a thread is fastened at the free end of the middle leg which thread when the IUD is in use is situated at least partially in the cervix or neck of the uterus and serves to permit easy removal of the IUD from the uterus.

Such IUD's are used for insertion into the uterine cavity of a woman, in which case, for example, a contraceptive, such as a copper coil or a product which has to be absorbed slowly in the body is fitted around or placed on the middle leg for the laterally directed projections of the IUD.

Such a device has been described in the US Patent no. 3,407,806 and which relates to an IUD, with a body which comprises a base portion with a pair of arcuate flanks and a projecting integral cylinderical connector. A strand is connected to the base portion, preferably by being imbedded during molding within the connector. However the strand has been molded for such a short distance in the IUD, that both ends of the strand need to be used and to tie the strand properly to the IUD it ought to be knotted.

In the US Patent no. 3,842,826 a device is described which is an intra uterine contraceptive device (IUD) and to which device is secured a flexible string with a relatively rigid molded line as stiffener and molded in place in the extruded stem portion. The free end of the string carries a small hard ball, molded thereon.

The cross pieces serve to hold the IUD in place in the uterine cavity after the IUD has been inserted into the uterus of a user, while a pulling element must be fitted near the free ends of the middle leg for the purpose of permitting easier removal of the IUD subsequently from the uterine cavity. IUD's must be removed from the uterine cavity after a short or longer period of time has elapsed following their insertion into the uterine cavity, for example when the inserted IUD is presumed to be no longer working in an optimum manner, or when complications such as undesirable infections occur. The removal of the IUD can take place by pulling the pulling element projecting out of the cervix of at least lying in the cervix, since the IUD itself generally lies too far up in the uterine cavity for it to be easily gripped with an instrument.

Providing a IUD with a pulling element made of a double thread fixed by means of a knot to the IUD is generally known. For this, the IUD can be provided with, for example, a hole or a narrowed part around which the pulling thread is fastened. It has however been found dat these pulling threads, which project through the passage of the neck of the womb into the vagina, are responsible for the occurrence of infections in the cervix or in the uterus. It has been found that a thread, and in particular a thread in which there is a knot, is a breading ground for the accumulation of bacteria, through the fact that these threads have a good adhesion surface for bacteria and the hollows between the threads and the knots are a well-protected place for the accumulation of bacteria. This can, of course, give rise to unpleasant consequences for the user.

The invention provides an improvement of the process for the manufacture of the IUD through the fact that it is characterized in that the IUD is an essentially T-shaped element with a middle leg being the middle projection with the thread running along the entire length of the IUD, that subsequently the ends of the thread near the topside of each IUD are cut off and that the IUD is molded of resiliently flexible plastic material and the thread is made of another plastic material with a higher fusion temperature.

From the European Patent Application EP-A-0 274 794 it is known that an IUD can be an essentially T-shaped element with a middle leg. This IUD comprises a wire being the pulling element, which is fastened through the middle leg via a small hole. This can cause serious accumulation of bacteria, as has been described above.

With the process according to the invention, a carrier or IUD is obtained with a pulling element which is connected very firmly to the IUD, as a result of which no breakage will occur when it has to be removed again from the uterus, and it is also possible in this way to fasten a single thread to the IUD without the use of knots or wholes, which means that possible sources of infection are greatly reduced.

It is preferable, after the IUD is manufactured by injection molding, to deform the end of the thread near the topside of the IUD. A better fastening of the thread to the IUD is obtained in this way.

In order to obtain a better connection of the thread to the IUD, the IUD with pulling thread can also be heated up again to such a temperature that the two materials from which the thread and the IUD are made fuse together and the loose end of the thread can also be heated in such a way that the end melts.

The invention also relates to a carrier or IUD of the type described in claims 4 to 7.

Since the thread according to the invention can now be single, and in particular can be made of a smooth plastic thread, the adhesion surface provides fewer possibilities for bacteria to adhere to it; the total adhesion surface for bacteria is also smaller, since a single thread is used and the chance of infection is thereby reduced accordingly, while the knot of the type always used hitherto in the thread to fasten this thread to an IUD is no longer present here, which hitherto was the greatest source of undesirable infection.

The invention will be explained in greater detail below with reference to a drawing, in which:
Figure 1 shows an IUD with a pulling element according to the invention;
Figure 2 shows in longitudinal section the IUD along line II-II from Figure 1;
Figure 3 shows in cross-section the IUD along line III-III from Figure 1.
Figure 4 shows a thread with several IUDs.
Figures 5, 6, 7 and 8 show in longitudinal section the IUD along line II-II from figure 1 according to another possibility of putting the thread inside the IUD.

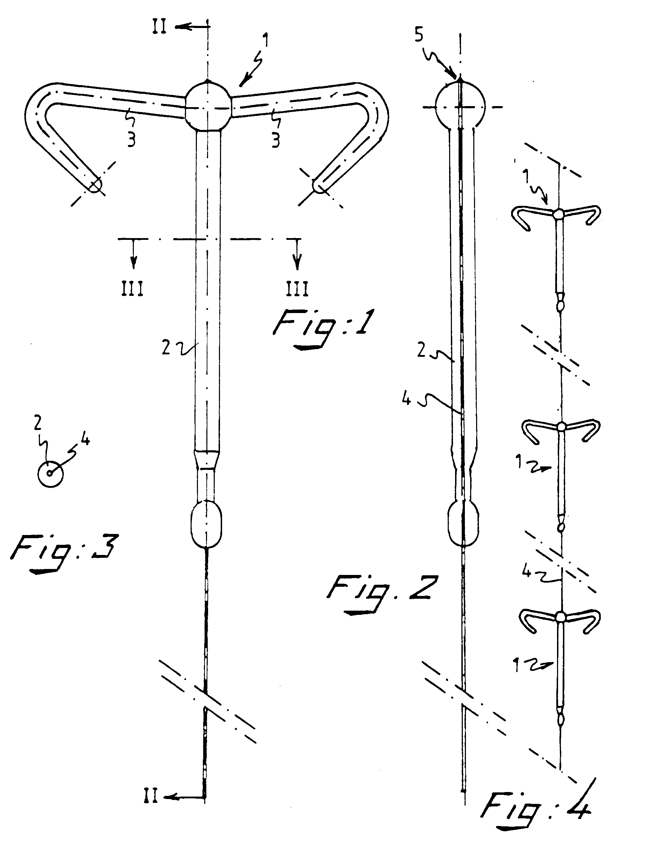

Figure 1 shows an IUD with pulling element according to the invention, which shows a simplified representation of an example of an embodiment of the IUD according to the invention.

The IUD illustrated in Figure 1 comprises a T-shaped carrier element 1 with a middle leg 2 and two cross legs 3. A coil or a product which is to be absorbed slowly by the body is placed on this middle leg 2. These cross legs 3 can also be designed in many other possible ways.

A thread 4 of, for example, polypropylene is fitted in the middle leg 2. This plastic thread 4 runs along the entire length through the middle leg 2. Since this thread 4 runs along the entire length, it is wedged in by the plastic of the middle leg 2 of the IUD cast around it.

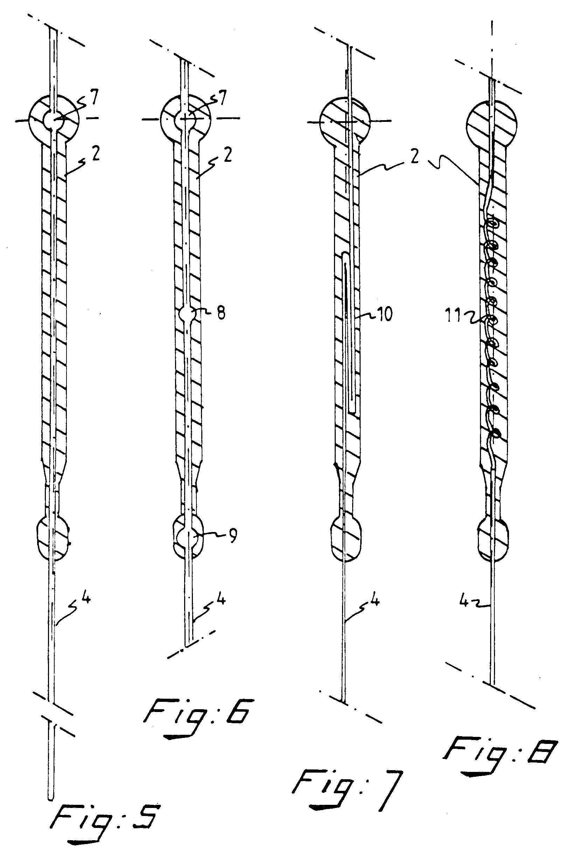

Figure 2 shows in longitudinal section, along the line II-II of Figure 1, the middle leg of the IUD. The pulling thread of plastic is preferably cut off near the end 5, at the point where it projects at the top side of the IUD. In another embodiment of the IUD according to the invention the thread is deformed near the top side, for example by temporarily heating it above the melting point of the plastic. Near the loose end 6, the thread is heated above the elastic limit, so that it does not have any sharp edges.

Figure 3 shows, finally, the IUD with pulling thread in cross-section along the line III-III from Figure 1.

Figure 4 shows a thread 4 with several IUDS 1.

A very simple fastening of a pulling thread is obtained in this way. Due to the fact that this thread is single and is made of a smooth plastic, the chance of infections of the cervix and/or the uterus occurring is very greatly reduced compared with the hitherto known IUD with a pulling element. First, the surface of the pulling thread is reduced at least by half, because this thread is single. Secondly, the use of a plastic thread means a reduction of infections, because a thread with smoother surface offers fewer possibilities for bacteria adhering to its surface. Thirdly, the result of leaving out a fastening of a knot or another fastening possibility in which small hollows are produced is a great reduction of the possibility of the accumulation of bacteria.

Figures 5, 6, 7 and 8 show other possibilities of incorporating a plastic thread inside the middle leg of the IUD. Figure 5 shows an IUD with a thread which has a thickness 7 which is incorporated in the top of the IUD. Figure 6 shows a cross-section of the IUD with a thread with several thicknesses, like knobs 7, 8, 9. Figure 7 shows in longitudinal section, along the line II-II figure 1, the middle leg of the IUD with a thread which is incorporated in the leg in a zigzag way 10. Figure 8 shows, finally, a possibility in which the incorporated thread is coiled inside the leg of the IUD. This thread can be coiled more than once 11.

## Claims

1. Process for the manufacture of an intra uterine device or IUD made of plastic material (1), provided with a pulling element, and a middle projection involving that first a thread (4) is placed in the injection mold for the manufacture of the IUD (1) in the lengthwise direction of the middle projection and that subsequently the mold is closed and the IUD (1) is produced by injection molding, after which the IUD (1) is removed from the mold, **characterized in that** the IUD is an essentially T-shaped element with a middle leg being the middle projection with the thread running along the entire length of the IUD, that subsequently the ends (5) of the thread (4) near the top-side of each IUD (1) are cut off and that the IUD is molded of resiliently flexible plastic material and the thread (4) is made of another plastic material with a higher fusion temperature.

2. Process according to claim 1 **characterized in that** on the same thread (4), more than one IUD (1) is produced by injection molding at an intermediate distance of eachother.

3. Process according to claim 1 or claim 2 **characterized in that** after the IUD (1) is produced by injection molding, the end (5) of the thread (4) near the topside of the IUD (1) is deformed.

4. IUD manufactured according to any of the processes of claim 1 to 3 **characterized in that** the IUD (1) is an essentially T-shaped element with a middle leg and a thread, acting as a pulling element, which thread is running along the entire length of the IUD and that it is molded of resiliently flexible plastic material such as poly-ethylene and the thread (4) is made of another plastic material with a higher fusion temperature such as nylon or poly-propylene.

5. Carrier for a contra-ceptive of the coil type known by the name of IUD, which carrier comprises an essentially T-shaped plastic element with a middle leg (2) on which a so called coil is wound, while cross pieces of the T-shape serve to hold the IUD (1) in place after the IUD (1) has been introduced into the uterus of a user, and a thread (4) is fastened at the free end of the middle leg (2) which thread (4) when the IUD (1) is in use is situated at least partially in the cervix or neck of the uterus and serves to permit easy removal of the IUD from the uterus, **characterized in that** the thread is single and the IUD is cast around the thread (4), that the thread runs along the entire length of the middle leg (2) and that the IUD is molded of resiliently flexible plastic material and the thread (4) is made of another plastic material with a higher fusion temperature.

6. Carrier according to claim 5 **characterized in that** the thread (4) is a single smooth thread (4), made of a plastic, for example nylon, poly-propylene or poly-urethane.

7. Carrier according to claim 5 **characterized in that** the thread (4) is at least partially fused with the middle leg (2).

## Patentansprüche

1. Verfahren für die Herstellung eines Intrauterinspessars oder IUP aus Kunststoff (1), versehen mit einem Zugteil und einer Mittenauskragung, wobei ein Faden (4) in eine Spritzgießform für die Herstellung des IUP in Längsrichtung der Mittenauskragung eingebracht, die Form anschließend geschlossen und der IUP (1) durch Spritzgießen hergestellt wird, wonach der IUP (1) aus der Form entfernt wird, dadurch gekennzeichnet, daß der IUP ein im wesentlichen T-förmiges Teil mit einem Mittelschenkel ist, welcher die Mittenauskragung mit dem über die ganze Länge des IUP verlaufenden Faden ist, daß nachträglich die Enden (5) des Fadens (4) in der Nähe der oberen Seite von jedem IUP (1) abgetrennt werden, und daß der IUP aus elastisch biegsamem Kunststoffmaterial geformt und der Faden (4) aus einem anderen Kunststoffmaterial mit einer höheren Schmelztemperatur hergestellt wird.

2. Verfahren gemäß Anspruch 1, dadurch gekennzeichnet, daß mit demselben Faden (4) mehr als ein IUP (1) durch Spritzgießen mit einem jeweiligen Zwischenabstand zueinander hergestellt wird.

3. Verfahren gemäß Anspruch 1 oder Anspruch 2, dadurch gekennzeichnet, daß, nachdem der IUP (1) durch Spritzgießen hergestellt wurde, das Ende (5) des Fadens (4) an der oberen Seite des IUP (1) verformt wird.

4. Ein IUP der, nach einem Verfahren gemäß einem der Ansprüche 1 bis 3 hergestellt wurde, dadurch gekennzeichnet, daß der IUP (1) ein im wesentlichen T-förmiges Teil mit einem Mittelschenkel und einem Faden ist, der als ein Zugteil wirkt, wobei der Faden über die gesamte Länge des IUP verläuft, und daß er aus elastisch biegsamen Kunststoffmaterial, wie z. B. aus Polyethylen, geformt ist und der Faden (4) aus einem anderen Kunststoffmaterial mit einer höheren Schmelztemperatur, z. B. Nylon oder Polypropylen, hergestellt wird.

5. Ein Träger für ein Kontrazeptivum von der Art einer Spirale, das unter dein Namen IUP bekannt ist, wobei der Träger ein im wesentlichen T-förmiges Kunststoffteil mit einem Mittelschenkel (2) aufweist, auf dein eine sog. Spirale gewickelt ist, während Querstücke der T-Form dazu dienen, den IUP am Ort zu halten, nachdem der IUP (1) in den Uterus einer Benutzerin eingeführt wurde, und an dem ein Faden (4) am freien Ende des Mittelschenkels (2) befestigt ist, wobei der Faden (4) bei Benutzung des IUP (1) mindestens teilweise in der Zervix oder im Hals der Gebärmutter liegt und zur mühelosen Entfernung des IUP aus der Gebärmutter dient, dadurch gekennzeichnet, daß der Faden einstückig und der IUP um den Faden (4) herum vergossen ist, daß der Faden über die gesamte Länge des Mittelschenkels (2) verläuft, und daß der IUP aus einem elastisch biegsamen Kunststoffmaterial geformt und der Faden (4) aus einem anderen Plastikmaterial mit einer höheren Schmelztemperatur hergestellt ist.

6. Ein Träger gemäß Anspruch 5, dadurch gekennzeichnet, daß der Faden (4) ein einstückiger glatter Faden (4) aus einem Kunststoff, z. B. Polypropylen oder Polyurethan, ist.

7. Ein Träger gemäß Anspruch 5, dadurch gekennzeichnet, daß der Faden (4) mindestens teilweise mit einem Mittelschenkel (2) verschmolzen ist.

## Revendications

1. Procédé de fabrication d'un dispositif intrautérin, ou DIU, fabriqué en matière plastique (1), muni d'un élément à tirer et d'une saillie centrale impliquant tout d'abord la mise en place d'un fil (4) dans le moule d'injection utilisé pour la fabrication du DIU (1), dans la direction longitudinale de la saillie centrale, puis la fermeture ultérieure du moule et la fabrication du DIU (1) par moulage par injection, caractérisé en ce que le DIU est un élément essentiellement en forme de T muni d'une tige centrale constituant la saillie centrale, le fil étant situé sur toute la longueur du DIU, en ce que les extrémités (5) du fil (4) à proximité du côté supérieur de chaque DIU sont coupés, et en ce que le DIU est moulé en matière plastique flexible élastique et que le fil (4) est fabriqué dans une autre matière plastique avec une température de fusion plus élevée.

2. Procédé selon la revendication 1 caractérisé en ce que sur le même fil (4), plusieurs DIU (1) sont fabriqués par moulage par injection, à distance intermédiaire l'un de l'autre.

3. Procédé selon la revendication 1 ou 2 caractérisé en ce que après fabrication du DIU par moulage par injection, l'extrémité (5) du fil (4) à proximité du bord supérieur du DIU (1) est déformée.

4. DUI fabriqué selon l'un quelconque des procédés des revendications 1 à 3 caractérisé en ce que le DIU (1) est principalement un élément en forme de T muni d'une tige centrale et d'un fil servant d'élément à tirer, ledit fil étant situé le long du DIU, sur toute sa longueur, en ce que le DIU est fabriqué en matière plastique flexible élastique telle que du polyéthylène, et que le fil (4) est fabriqué dans une autre matière plastique avec une température de fusion plus élevée, telle que du nylon ou du polypropylène.

5. Support pour contraceptif du type à enroulement connu sous le nom de DIU, ledit support comportant un élément en plastique essentiellement en forme de T muni d'une tige centrale (2) sur laquelle est fixée ledit enroulement, les éléments transversaux de la forme en T servant à maintenir le DIU (1) en place après insertion du DIU (1) dans l'utérus de l'utilisatrice, un fil (4) étant fixé à l'extrémité libre de la tige centrale (2), ledit fil (4), lorsque le DIU est en place, étant situé au moins partiellement dans le col de l'utérus et permettant un retrait facile du DIU de l'utérus, caractérisé en ce que le fil est simple et le DIU est moulé autour du fil (4), en ce que le fil est situé le long de la tige centrale (2) sur toute sa longueur, et en ce que le DIU est moulé dans une matière plastique flexible élastique et le fil (4) fabriqué dans une autre matière plastique avec une température de fusion plus élevée.

6. Support selon la revendication 5 caractérisé en ce que le fil (4) est un fil lisse simple (4) en matière plastique, par exemple du nylon, du polypropylène ou du polyuréthane.

7. Support selon la revendication 5 caractérisé en ce que le fil (4) est au moins partiellement fondu avec la tige centrale (2).
